# EUROPEAN PATENT SPECIFICATION

(11) **EP 2 968 218 B1**
(45) Date of publication and mention of the grant of the patent: **07.07.2021**
(21) Application number: 14718177.0
(22) Date of filing: 13.03.2014
(51) Int. Cl.: A61K 31/121, A61K 31/198, A61K 31/277, A61P 25/16, A61P 43/00, A61K 9/00, A61K 9/08

(54) **COMBINATION FOR THE TREATMENT OF PARKINSON'S DISEASE**
KOMBINATION ZUR BEHANDLUNG DER PARKINSON-KRANKHEIT
COMBINAISON POUR LE TRAITEMENT DE LA MALADIE DE PARKINSON

(30) Priority: 13.03.2013 US 201361779357 P
(43) Date of publication of application: 20.01.2016
(60) Divisional application: 21176202.6
(73) Proprietor: Neuroderm Ltd, 74036 Ness Ziona (IL)
(72) Inventor: YACOBY-ZEEVI, Oron, 6094600 Bitsaron (IL)
(74) Representative: Hoffmann Eitle
(86) International application number: PCT/IL2014/050261
(87) International publication number: WO 2014/141261

(56) References cited:
- WO-A1-2012/066538
- US-A1- 2010 298 428
- NYHOLM D ET AL: "Levodopa infusion combined with entacapone or tolcapone in Parkinson disease: a pilot trial.", EUROPEAN JOURNAL OF NEUROLOGY : THE OFFICIAL JOURNAL OF THE EUROPEAN FEDERATION OF NEUROLOGICAL SOCIETIES JUN 2012, vol. 19, no. 6, June 2012 (2012-06), pages 820-826, XP002724127, ISSN: 1468-1331
- "Pharmacokinetics of Levodopa/Carbidopa Infusion With and Without Oral Catechol-O-methyl Transferase (COMT) Inhibitors (DuoCOMT)", INTERENET , January 2010 (2010-01), XP002724128, Retrieved from the Internet: URL:http://clinicaltrials.gov/ct2/show/NCT 00906828 [retrieved on 2014-05-09]
- AbbVie Limited: "Duodopa intestinal gel", INTERNET , 24 October 2013 (2013-10-24), XP002724129, Retrieved from the Internet: URL:https://www.medicines.org.uk/emc/medic ine/20786/SPC/Duodopa+intestinal+gel/#COMP OSITION [retrieved on 2014-05-09]
- NORD MARIA ET AL: "The effect of peripheral enzyme inhibitors on levodopa concentrations in blood and CSF.", MOVEMENT DISORDERS : OFFICIAL JOURNAL OF THE MOVEMENT DISORDER SOCIETY 15 FEB 2010, vol. 25, no. 3, 15 February 2010 (2010-02-15), pages 363-367, XP002724130, ISSN: 1531-8257
- None

## Description

### TECHNICAL FIELD

The present invention provides a combination for the treatment of Parkinson's disease by subcutaneous administration of levodopa and carbidopa, concomitantly with oral administration of entacapone.

### BACKGROUND

Parkinson's disease is a degenerative condition characterized by reduced concentration of the neurotransmitter dopamine in the brain. Levodopa (L-dopa or L-3,4-dihydroxyphenylalanine) is an immediate metabolic precursor of dopamine that, unlike dopamine, is able to cross the blood-brain barrier, and is most commonly used for restoring the dopamine concentration in the brain. For the past 40 years, levodopa has been remained the most effective therapy for treatment of Parkinson's disease.

However, levodopa has a short half-life in plasma that, even under best common current standard of care, results in pulsatile dopaminergic stimulation. Long-term therapy is therefore complicated by motor fluctuations and dyskinesia that can represent a source of significant disability for some patients. A therapeutic strategy that could ultimately deliver levodopa/dopamine to the brain in a more continuous and physiologic manner would provide the benefits of standard levodopa with reduced motor complications and is much needed by patients suffering from Parkinson's disease and other neurological or movement disorders (Olanow, Mov. Dis., 2008, 23(Suppl. 3), S613-S622). Sustained-release oral levodopa formulations have been developed but, at best, such preparations have been found to be no more efficacious than standard tablets. Continuous administration of levodopa by intraduodenal administration or infusion has also been attempted by using ambulatory pumps or patches. Such treatments, especially intraduodenal, are extremely invasive and inconvenient.

The metabolic transformation of levodopa to dopamine is catalyzed by the aromatic L-amino acid decarboxylase enzyme, a ubiquitous enzyme with particularly high concentrations in the intestinal mucosa, liver, brain and brain capillaries. Due to the extracerebral metabolism of levodopa, it is necessary to administer large doses of levodopa leading to high extracerebral concentrations of dopamine that cause unwanted side effects in some of the patients. Therefore, levodopa is usually administered concurrently with oral administration of a dopa decarboxylase inhibitor, such as carbidopa or benserazide, which reduces by 60-80% the levodopa dose required for a clinical response, and thus prevents certain of its side effects by inhibiting the conversion of levodopa to dopamine outside of the brain.

Additional extracerebral metabolic pathways of levodopa are through monoamine oxidase (MAO), or catechol-O-methyl transferase (COMT) which converts levodopa to 3-methoxy-4-hydroxy-L-phenylalanine (3-O-methyldopa, 3-OMD), a metabolite that competes with levodopa in crossing the blood-brain barrier. Therefore, MAO inhibition by, e.g., moclobemide, rasagiline, selegiline or safinamide, or COMT inhibition by, e.g., entacapone or tolcapone, improves the efficacy of levodopa. Entacapone exhibits short biphasic elimination, with a β-phase half-life of about 0.4 to about 0.7 hours and a γ-phase half-life of about 2.4 hours and is therefore typically administered six to eight times per day. Tolcapone binds to the catalytic center of COMT in both peripheral and central nervous systems with greater affinity than any of the three catecholamine, including levodopa, and consequently prevents the 3-O-methylation of levodopa by COMT. Tolcapone thus improves the bioavailability and reduces the clearance of levodopa and subsequently dopamine from the central nervous system.

The efficacy of levodopa's metabolic pathways inhibitors, when concomitantly administered with levodopa, in increasing the level of the latter in the blood is well documented, and various formulations of levodopa together with such inhibitors have been disclosed. For example, currently available oral drugs include SINEMET® and sustained-release SINEMET® CR tablets that include levodopa and carbidopa, as well as MADOPAR® tablets containing levodopa and benserazide. Additional formulations of levodopa and carbidopa for subcutaneous, preferably continuous, administration are disclosed in International Publication Nos. WO 2010/134074 and WO 2012/066538.

An additional oral drug is STALEVO® tablets containing levodopa, carbidopa and entacapone, and formulations of these ingredients, e.g., for subcutaneous administration, are disclosed in the aforesaid WO 2012/066538, which further discloses oral administration of SINEMET® and concomitant continuous subcutaneous administration of either entacapone or entacapone and carbidopa.

Nyholm et al. (European Journal of Neurology, 2012, 19, 820-826) shows that oral administration of 200 mg entacapone and concomitant intraintestinal infusion of levodopa and carbidopa at a dosage equal to 80% of the optimized dose of a patient, allows maintaining a blood level of levodopa that is substantially the same as that achieved by administration of the optimized dose of levodopa and carbidopa, without entacapone. In other words, Nyholm *et al.* shows that oral administration of entacapone allows reducing the initial dose of levodopa and carbidopa administered by 20% and still maintain the desired blood level of levodopa.

Yet, the various formulations disclosed do not provide the optimal treatment of Parkinson's disease due to still relatively high level of side effects and levodopa blood level fluctuations. There is thus an ongoing and urgent need for methods for treatment of movement disorders such as Parkinson's, that will provide patients with a constant blood levodopa level that will lead to constant dopaminergic stimulation in the brain, and at the same time limit the side effects caused by high peripheral levodopa concentration resulting from high dose levodopa administration.

### SUMMARY OF INVENTION

The present invention provides carbidopa, levodopa and entacapone for use as a combination in the treatment of Parkinson's disease, wherein the carbidopa and the levodopa are formulated as a sole parenteral composition for continuous subcutaneous administration, and said entacapone is formulated as an oral composition and is administered at a dose of 400, 450, 500, 550 or 600 mg twice or three times a day.

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body by therapy.

### BRIEF DESCRIPTION OF THE FIGURES

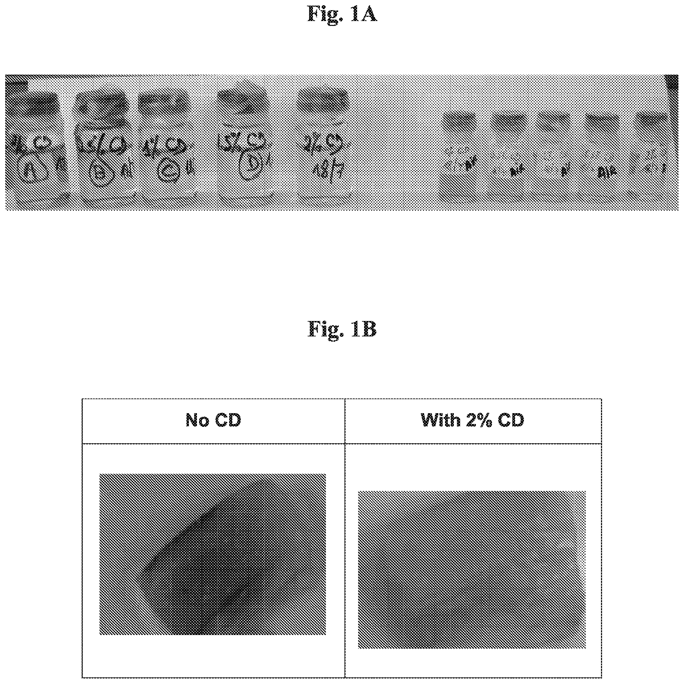

**Figs. 1A-1B** show the effect of carbidopa on the stability of levodopa *in vitro* and *ex vivo:* **1A**. 6% by weight levodopa and arginine solution with various concentrations (2, 1.5, 1, 0.5%) of carbidopa or no carbidopa were tested for physical stability *in vitro.* The results show that carbidopa prevented dark yellow color formation in the presence of air, in a dose related manner (small vials at the right hand side), and in the absence of air (with N₂ in the head space) 0.5% carbidopa was sufficient to inhibit this color formation (large vials in the left hand side of the figure). **1B.** 7% by weight levodopa and arginine solution, with or without 2% carbidopa by weight, continuously administered in to the subcutaneous tissue of a 5x5cm fresh, full-thickness pig skin. The right hand side depicts the inhibition of oxidation with the use of a levodopa formulation that includes carbidopa.

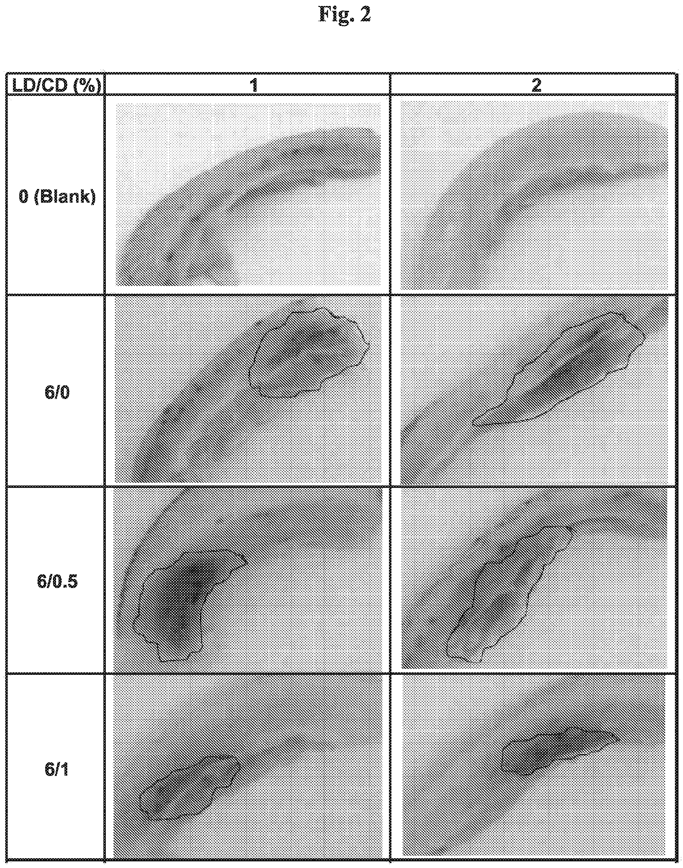

**Fig. 2** shows that the presence of 1% carbidopa in a levodopa solution reduces the severity and extent of local levodopa dependent subcutaneous toxicity in the pig.

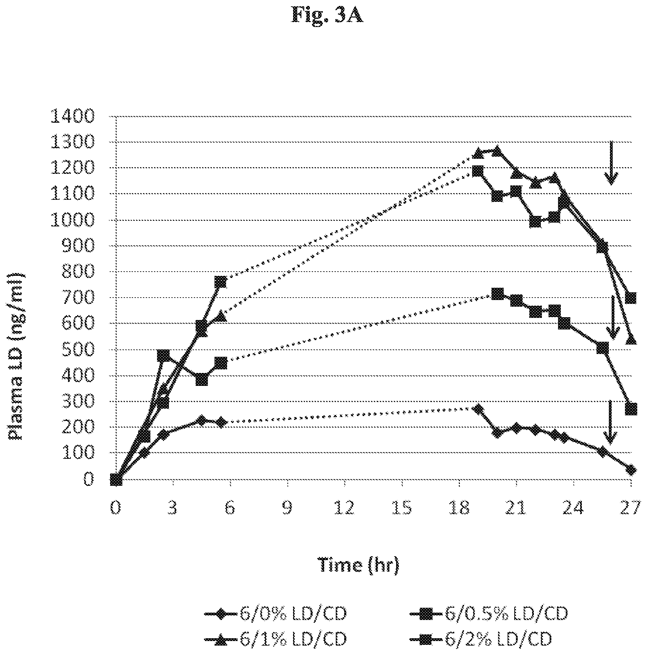

**Figs. 3A-3C** show the effect of carbidopa on the pharmacokinetics of levodopa in the pig. **3A:** the plasma concentration of levodopa following continuous subcutaneous administration of 6% levodopa with various amounts of carbidopa. **3B:** The correlation between plasma steady state concentrations of levodopa, obtained following continuous subcutaneous administration of levodopa/carbidopa formulations and the formulation concentration of carbidopa. **3C:** The correlation between plasma steady state concentration of carbidopa following continuous subcutaneous administration of levodopa/carbidopa formulations and the formulation concentration of carbidopa.

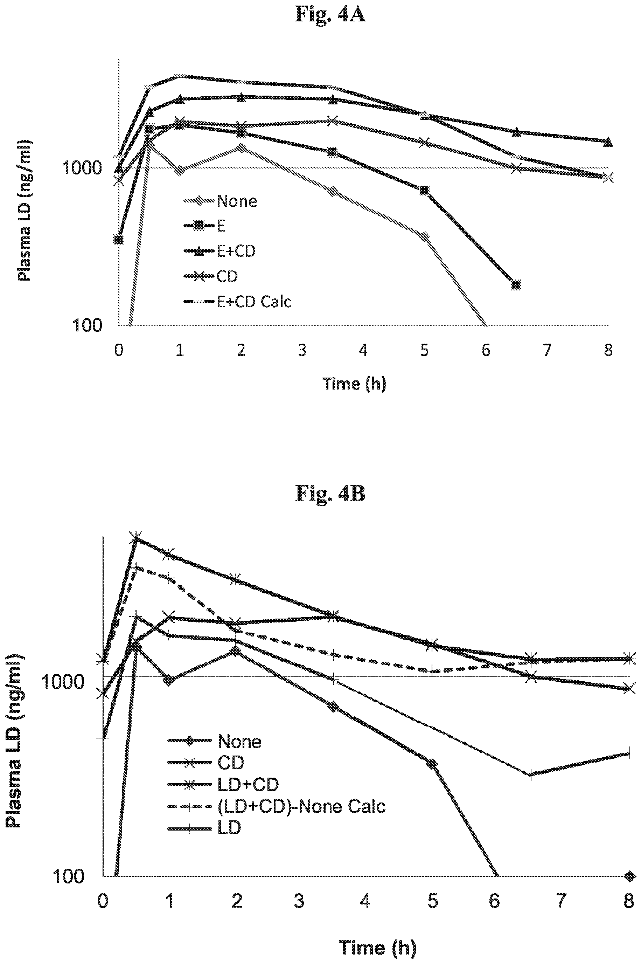

**Figs. 4A-4B** (Reference) show the effect of continuous subcutaneous (SC) entacapone (E) and/or carbidopa (CD) (40 mg/24 h) administration on the plasma concentrations of levodopa (LD, ng/ml) following oral administration of Sinemet (100/25 levodopa/ carbidopa) in pigs (**4A**); and the effect of continuous SC CD (40 mg/24 h) and/or LD (140 mg/24 h) administration on the plasma concentrations of LD following oral administration of Sinemet in pigs (**4B**).

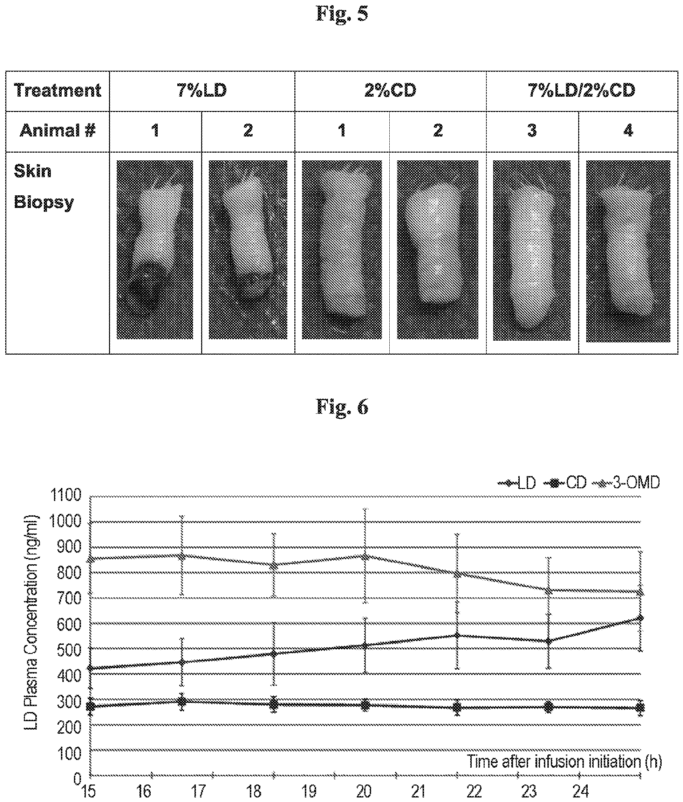

**Fig. 5** shows the effect of carbidopa on the local subcutaneous toxicity of levodopa following 24 h continuous subcutaneous administration, at 0.16 ml/h, in pigs.
**Fig. 6** show the effect of oral administration of entacapone (200 mg every 2 h) on the plasma concentrations of levodopa (LD), carbidopa (CD), and 3-O-methyldopa (3-OMD) during continuous SC administration of levodopa (360 mg/24 h) and carbidopa (90 mg/24 h) in human volunteers.

### DETAILED DESCRIPTION OF THE INVENTION

As stated above, the concomitant administration of levodopa and an active agent capable of inhibiting a metabolic pathway of levodopa is well known, and one of the commercially available drugs for treatment of Parkinson's disease is STALEVO® that, in fact, comprises a combination of levodopa, carbidopa and entacapone. Nevertheless, despite of the advantages associated with a joint oral administration of the three ingredient, this specific route of administration has several drawbacks because oral administration results in fluctuations of blood levodopa level; and entacapone, when present in one solution with carbidopa, e.g., after dissolution of a tablet in the gastrointestinal tract), reduces carbidopa's bioavailability. This is clearly indicated in Ahtila et al. (Clin Neuropharmacol., 1995, 18(1), 46-57), demonstrating that high doses of entacapone (400 mg and 800 mg), concomitantly orally administered with levodopa and carbidopa, decrease the total amount of carbidopa in blood but do not affect the time to maximal concentration (Tₘₐₓ) of carbidopa. Ahtila *et al.* also show that the highest increase in the total amount of levodopa caused by the addition of entacapone to oral administration of levodopa and carbidopa was about 33% (after 400 mg dose of entacapone).

The aforesaid suggests that it might be beneficial to administer carbidopa separately from entacapone, i.e., using either different administration routes or by administering them in two different locations of the patient's body. Indeed, as already shown by the present inventors and shown in WO 2012/066538, oral administration of Sinemet® (levodopa/carbidopa, 100/25 mg) and concomitant subcutaneous administration of either entacapone or a combination of entacapone and carbidopa (formulated as different compositions) resulted in increased blood levels of levodopa. As specifically shown, entacapone and carbidopa, when continuously subcutaneously co-administered with Sinemet®, act in synergy on the plasma pharmacokinetics of levodopa.

In another approach disclosed in the aforesaid Nyholm *et al.,* entacapone is orally administered while a combination of levodopa and carbidopa is administered concomitantly by intraintestinal infusion, and as shown, oral administration of entacapone allows reducing the initial dose of levodopa and carbidopa administered by 20% while maintaining the desired blood level of levodopa. According to Nyholm *et al.,* the administration route disclosed may allow an increase of up to 33% in the level of levodopa.

It has now been found, in accordance with the present invention, that continuous subcutaneous administration of a composition comprising both levodopa and carbidopa, and concomitant oral administration of entacapone to Parkinson's patients unexpectedly increases the blood level of levodopa by more than 40%, more particularly from about 420 ng/ml to about 620 ng/ml, while maintaining a constant blood level of levodopa with no substantial fluctuations, and therefore allows significantly reducing the initial dose of levodopa administered to Parkinson's patients and subsequently reducing the side effects of the drug. Moreover, the experimental data provided herein show that the subcutaneous administration of a sole composition comprising levodopa and carbidopa have additional benefits compared with separate administrations thereof due to reduced local side effects caused by levodopa when administered together with carbidopa.

These findings show that oral administration of entacapone concomitantly with continuous subcutaneous administration, of levodopa and carbidopa leads to levodopa blood level that is remarkably higher than those shown in any one of the prior art utilizing a different administration regimen, suggesting that the administration regimen demonstrated herein allows to utilize to the fullest the inhibitory potentials of both inhibitors.

The present invention thus provides a strategy for treatment of Parkinson's disease, comprising continuous subcutaneous administration of a combination of levodopa and carbidopa concomitantly with oral administration of entacapone. Such a strategy allows providing high plasma levodopa level thereby continuous stimulation of the dopaminergic system in the brain, and may allow decreasing the entacapone oral dosing regimen from 5-8 times/day to 2-3 times/day, and/or reduce daily dose of levodopa and/or entacapone. In one embodiment, the neurological disease to be treated according to this strategy is Parkinson's disease.

In a particular embodiment, the dopa decarboxylase inhibitor used according to the treatment strategy disclosed herein is carbidopa.

The term "catechol-O-methyl transferase (COMT) inhibitor" as used herein refers to an agent capable of inhibiting the degradation of levodopa to 3-O-methyldopa (3-OMD) by catechol-O-methyl transferase and thus prolonging the half-life of levodopa in the circulation. An example of COMT inhibitor is entacapone.

In a particular embodiment, the combination of levodopa and carbidopa is formulated as a sole pharmaceutical composition that is continuously subcutaneously administered to the treated individual; and entacapone is formulated as a pharmaceutical composition orally administered to said individual (herein *"**the oral composition***").

The term "pharmaceutical composition" as used herein refers to a composition comprising at least one active component as disclosed herein formulated together with one or more pharmaceutically acceptable carriers or pharmaceutically acceptable excipients. The terms "pharmaceutically acceptable carrier" and "pharmaceutically acceptable excipient" as used herein refer to any and all solvents, dispersion media, preservatives, antioxidants, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions. "Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, safety and purity standards as required by, e.g., the U.S. FDA Office of Biologics standards.

The parenteral composition is administered continuously subcutaneously. Continuous administration of the parenteral composition may be accomplished using, e.g., an infusion pump, or a dermal patch, namely, a device suitable for transdermal or subcutaneous administration of the parenteral composition, i.e., capable of delivering said composition through the skin or mucous membrane into the bloodstream of a patient. Dermal patches suitable for use according to the method of the invention may have one or more compartments containing the parenteral composition or compositions to be administered, and are described, e.g., in the aforesaid WO 2012/066538.

The weight ratio between the levodopa and carbidopa, administered according to the method of the present invention may be any weight ratio that, together with entacapone concomitantly administered, would be sufficient to maintain therapeutic plasma levels of levodopa, when said levodopa and carbidopa are formulated as a sole parenteral composition. In certain aspects, the levodopa: carbidopa weight ratio is in a range of 20:1 to 1:1.

In certain embodiments, the parenteral composition further comprises arginine. According to particular such embodiments, the parenteral composition comprises carbidopa and levodopa in an overall molar ratio to arginine of 1:2 to 1:3.5. As previously shown by the present inventors and disclosed in WO 2010/134074 and WO 2012/066538, such compositions are significantly more stable than compositions where no arginine is used, or compositions where basic amino acid other than arginine, e.g., lysine or histidine, are used.

In some particular such embodiments, the parenteral composition according to the method of the invention comprises (i) arginine, 0.1% to 2% by weight carbidopa, and 4% to 8% by weight levodopa; or (ii) arginine, 0.6% to 1.5% by weight carbidopa, and 6% by weight levodopa. Such compositions may be administered at a rate of 0.1 to 1000 µl/hour/site; or at a volume of 2 to 10 ml/24hour/site, e.g., 4 to 6 ml/24hour/site; or at a dose of 80 to 800 mg levodopa/day and 20 to 200 mg carbidopa/day; or at a rate of 240 to 360 mg levodopa and 60 to 90 mg carbidopa/day/site. More specific such parenteral compositions are administered subcutaneously.

In other particular such embodiments, the parenteral composition comprises (i) arginine, 1% to 4% by weight carbidopa, and 6% to 16% by weight levodopa; or (ii) arginine, 1.5% to 2.5% by weight carbidopa, and 12% by weight levodopa. Such compositions may be administered at a rate of 0.2 to 2000 µl/hour/site; or at a volume of 10 to 24 ml/24hour/site, e.g., 12 to 16 ml/24hour/site; or at a dose of 600 to 4000 mg levodopa/day and 60 to 500 mg carbidopa/day; or at a rate of 800 to 1600 mg levodopa and 200 to 400 mg carbidopa/day/site.

In certain particular embodiments, the parenteral composition according to the method of the invention comprises arginine, levodopa and carbidopa, wherein the parenteral composition has levodopa:arginine molar ratio selected from 1:1.5 to 1:2.5, respectively, and a pH of 8.5 to 10, i.e., 8.5, 8.6, 8.7, 8.8, 8.9, 9.0, 9.1, 9.2, 9.3, 9.4, 9.5, 9.6, 9.7, 9.8, 9.9 or 10, at 25°C. Such compositions may comprise, e.g., 1% to 20% or more, by weight, carbidopa, preferably at least 1%, 2%, 4% or 6% by weight carbidopa; or at least 1%, preferably 2%, 3%, 4%, 5% or 6%, by weight levodopa. More particular such compositions may further comprise a pharmaceutically acceptable excipient such as N-methylpyrrolidone, polyvinylpyrrolidone, propylene glycol, or a combination thereof, and may still further comprise water. In specific embodiments, such parenteral compositions have a pH of 8.5 to 9.8 at 25°C.

In certain particular embodiments, the parenteral composition according to the method of the invention comprises arginine, carbidopa, at least about 4% by weight levodopa, and optionally meglumine, wherein the parenteral composition has a pH of 9.1 to 9.8, i.e., 9.1, 9.2, 9.3, 9.4, 9.5, 9.6, 9.7 or 9.8, at 25°C. Such compositions may have a molar ratio of active components, i.e., carbidopa and levodopa, to arginine of 1:1.8 to 1:3.5, or 1:2.2 to 1:2.5. More particular such compositions comprise 4% to 12%, or 5% to 30%, by weight levodopa, and/or 1% to 6%, or 1% to 2%, by weight carbidopa.

In some embodiments, the parenteral composition comprises arginine, carbidopa, and at least about 4% by weight levodopa, as defined hereinabove, and further comprises meglumine. Certain particular such compositions are those wherein the molar ratio of active components to the arginine is 1:1.1 to 1:1.9, and/or the molar ratio of active components to the meglumine is 1:0.3 to 1:1.2, 1:0.3 to 1:1.5, 1:0.4, or 1:1.1. Other particular such compositions are those comprising 2.0% to 11% by weight meglumine, and/or 10% to 35% by weight arginine.

In more particular embodiments, the parenteral composition comprises arginine, carbidopa, at least 4% by weight levodopa, and optionally meglumine, as defined in any one of the embodiments above, and further comprises at least one agent capable of inhibiting the formation of oxidation products, e.g., ascorbic acid or a pharmaceutically acceptable salt thereof such as sodium ascorbate, calcium ascorbate, potassium ascorbate, ascorbyl palmitate, or ascorbyl stearate, L-cysteine, N-acetylcysteine (NAC), gluthatione (GSH), Na₂-EDTA, Na₂-EDTA-Ca, or a combination thereof.

According to the present invention, parenteral compositions comprising arginine, carbidopa, at least 4% by weight levodopa, and optionally meglumine, as defined in any one of the embodiments above, may further comprise sodium bisulfite.

In certain specific embodiments, the parenteral composition according to the method of the present invention comprises 6% by weight levodopa, 0.6% to 1.4% by weight carbidopa, 15% to 16% by weight arginine, and 0.5% by weight ascorbic acid; may further comprise 0.4% by weight L-cysteine and/or 0.5% by weight NAC; and has a pH of 9.4 to 9.6.

In some aspects, the parenteral composition comprises about 12% to about 15% by weight levodopa, about 1.2% to 4% by weight carbidopa, 32% to 42% by weight of either arginine or meglumine; and 1.0% to 1.3% by weight sodium ascorbate; may further comprise 0.1% to 0.5% by weight L-cysteine and/or NAC and/or cysteine-HCl; and has a pH of 9.6 to 9.8.

In some aspects, a parenteral composition having any one of the various compositions defined above is administered concomitantly with an oral composition comprising entacapone. Entacapone may be administered at a dosage of, e.g., 10 to 1600 mg per day, 50 to 400 mg per day, 100 to 600 mg per day, 400 to 1200 mg per day, 1000 to 1400 mg per day, or 1200 mg to 1600 mg per day. The oral composition may be administered 1, 2, 3, 4 or 5 times a day. In particular embodiments, entacapone is administered at a lower frequency than presently recommended, e.g., two to three times per day, or in a lower daily dosage amount.

In certain embodiments, the oral composition comprises entacapone, and it is administered concomitantly with the parenteral composition at a dose 400, 450, 500, 550 or 600 mg, twice or three times a day. In more particular such embodiments, the oral composition is administered at a dose of 400 mg, twice a day.

Oral compositions of entacapone may be in any suitable form. For example, the oral composition may be formulated as a pill, hard or soft capsule, tablet, troches, lozenges, aqueous or oily suspensions, dispersible powders or granules, emulsions, syrups or elixirs. The oral formulation may comprise, e.g., 200 mg to 600 mg, 50 mg to 200 mg, or 100 mg, of entacapone per dose. In some cases, the oral formulation may be a controlled release formulation, i.e., formulated for controlled (sustained, extended, or prolonged) or delayed release of entacapone.

The various pharmaceutical compositions utilized according to the method of the invention may be prepared by conventional techniques, e.g., as described in Remington: The Science and Practice of Pharmacy, 19th Ed., 1995. The compositions can be prepared, e.g., by uniformly and intimately bringing the active component/components into association with a liquid carrier, a finely divided solid carrier, or both, and then, if necessary, shaping the product into the desired formulation. The compositions may be in liquid, solid or semisolid form and may further include pharmaceutically acceptable fillers, carriers, diluents or adjuvants, and other inert ingredients and excipients. In one embodiment, the pharmaceutical composition of the present invention is formulated as nanoparticles.

The parenteral compositions utilized may be prepared by mixing levodopa and carbidopa optionally together with arginine and/or meglumine and/or one or more antioxidants, in amounts as disclosed above, to form a powder mixture. Water may be added to the mixture to form a suspension. The suspension may be heated, e.g., to about 60-90°C, more particularly to about 72±5°C, e.g., by adding pre-heated water and/or by placing the mixture in a hot (e.g., 72±5°C) water bath for a sufficient time period, e.g., for about 3 minutes, about 5 minutes, about 10 minutes or more, to form a solution, with optional stirring, and cooling the solution to form the composition. N₂ may be provided the head space of the container. For example, the mixture can be removed from the hot water bath and cooled to room temperature (RT), and an antioxidant may then be added under N₂ atmosphere and subsequent stirring. A preparation such as that above, e.g., where levodopa, carbidopa, and arginine are mixed together as powders first, and a suspension with water is then formed and heated, may result in a more stable solution as compared to a preparation that includes a step wise preparation of individual water suspensions of ingredients and later combination.

The parenteral compositions may be sterilized, e.g., using 0.2 µM filters such as filters with nylon or polyvinylidene difluoride (PVDF) membranes. In some embodiments, the compositions have fewer undesirable by-products, e.g., toxic by-products, or contaminants, e.g., hydrazine, when carbidopa and levodopa are present at the same time, and when prepared using certain antioxidants, e.g., ascorbic acid or salts thereof, rather than others, e.g., sodium bisulfite. In other embodiments, the compositions have fewer undesirable by-products when pre-heated water is added as disclosed above, as compared to formulations prepared without the addition of pre-heated water. In further embodiments, the levodopa and/or carbidopa may not dissolve unless the preparation procedure described above is used. Such preparations as described above may provide more stable formulations as compared to formulations prepared without adding hot water or heating.

Oral compositions of entacapone may be prepared according to any method known in the art for the manufacture of pharmaceutical compositions and may further comprise one or more ingredients selected from sweetening agents, flavoring agents, coloring agents and preserving agents in order to provide pharmaceutically elegant and palatable preparations. Tablets contain the active component, i.e., the COMT inhibitor, in admixture with non-toxic pharmaceutically acceptable excipients, which are suitable for the manufacture of tablets. These excipients may be, e.g., inert diluents such as calcium carbonate, sodium carbonate, lactose, calcium phosphate, or sodium phosphate; granulating and disintegrating agents, e.g., corn starch or alginic acid; binding agents, e.g., starch, gelatin or acacia; and lubricating agents, e.g., magnesium stearate, stearic acid, or talc. The tablets may be either uncoated or coated utilizing known techniques to delay disintegration and absorption in the gastrointestinal tract and thereby provide a sustained action over a longer period. For example, a time delay material such as glyceryl monostearate or glyceryl distearate may be employed. They may also be coated using the techniques described in the US Patent Nos. 4,256,108, 4,166,452 and 4,265,874 to form osmotic therapeutic tablets for control release. The oral compositions may also be in the form of oil-in-water emulsion.

The oral compositions may be formulated for either immediate or controlled release of entacapone. Such controlled release compositions may be formulated as controlled-release matrix, e.g., as controlled-release matrix tablets in which the release of a soluble active agent is controlled by having the active diffuse through a gel formed after the swelling of a hydrophilic polymer brought into contact with dissolving liquid (*in vitro*) or gastro-intestinal fluid (*in vivo*). Many polymers have been described as capable of forming such gel, e.g., derivatives of cellulose, in particular the cellulose ethers such as hydroxypropyl cellulose, hydroxymethyl cellulose, methylcellulose or methyl hydroxypropyl cellulose, and among the different commercial grades of these ethers are those showing fairly high viscosity. In other embodiments, the compositions comprise the active component formulated for controlled release in microencapsulated dosage form, in which small droplets of entacapone are surrounded by a coating or a membrane to form particles in the range of a few micrometers to a few millimeters.

Other contemplated formulations are depot systems, based on biodegradable polymers, wherein as the polymer degrades, the active component is slowly released. The most common class of biodegradable polymers is the hydrolytically labile polyesters prepared from lactic acid, glycolic acid, or combinations of these two molecules. Polymers prepared from these individual monomers include poly (D,L-lactide) (PLA), poly (glycolide) (PGA), and the copolymer poly (D,L-lactide-co-glycolide) (PLG).

The treatment strategy disclosed herein is aimed at increasing the half-life of administered levodopa and substantially reducing the pulsatility of plasma levels thereof, by co-administering levodopa and carbidopa, and substantially constantly inhibiting COMT activity, thus providing the individual treated with a constant plasma levodopa level that will lead to constant dopaminergic stimulation in the brain, and at the same time limiting the side effects caused by high plasma levodopa levels resulting from high dose levodopa administration. Contemplated administration of levodopa, carbidopa and entacapone according to the method of the present invention can typically be carried out over a defined time period, e.g., days, weeks, months and even years, depending on the state of the patient, and as deemed appropriate by the practitioner.

In another aspect, the present invention relates to carbidopa levodopa or a pharmaceutically acceptable salt thereof, and entacapone for use as a combination in treatment of Parkinson's disease, wherein carbidopa and levodopa are formulated, as a combination, as a continuous subcutaneous composition(s), and said entacapone is formulated as an oral composition.

In a particular such aspect, the invention relates to carbidopa, levodopa and entacapone for use as a combination in the treatment of Parkinson's disease, wherein the carbidopa and the levodopa are formulated as a sole parenteral composition as defined above, and said entacapone is formulated as an oral composition as defined above.

Particular such parenteral compositions are suitable for continuous administration.

Also contemplated herein is a kit comprising: (i) a parenteral composition as defined above, preferably continuous parenteral administration, said parenteral composition comprising carbidopa, levodopa, and arginine; (ii) an oral composition as defined above, i.e., a pharmaceutical composition formulated for oral administration, said oral composition comprising entacapone; and (iii) instructions for concomitant administration of the pharmaceutical compositions for the treatment of Parkinson's disease.

The parenteral composition included in the kit disclosed may be liquid or a lyophilized powder that can be reconstituted into a liquid formulation, or may form part of a dermal patch, and/or may be designed for continuous administration by subcutaneous administration. The oral composition included in the kit may be formulated for either immediate or controlled release of entacapone and may be in any one of the forms described above.

In certain embodiments, either the parenteral or oral composition included in the kit disclosed, or both, may be provided in a container(s), i.e., a pre-filled cartridge(s), suitable for use by a patient or a physician. For example, provided herein is a kit comprising a prefilled cartridge containing a parenteral liquid composition comprising carbidopa, levodopa and arginine; and an oral composition, e.g., one or more tablets or pills, of entacapone; and optionally instructions for use.

The invention now being generally described, will be more readily understood by reference to the following examples.

### EXAMPLES

### Example 1. Preparation of solutions/formulation for subcutaneous administration

A. A 2% carbidopa solution/formulation was prepared by adding pre-heated 0.1% Na-bisulfite solution to carbidopa [ASSIA Ltd.]. Arginine (Merck) was added to obtain a final molar ratio of 1:1.2 CD (carbidopa):Arg(arginine). The mixture was stirred at 60°C until complete dissolution was obtained. Heating was stopped and the preparation was allowed to cool down to RT; pH of 8.5. Solution was filtered using a sterile 0.22 µM PVDF membrane.
B. A 10% tolcapone solution/formulation was prepared as follows: a solution containing 10% tolcapone was prepared by adding the respective amount of H₂O to tolcapone (Synfine Research), slowly adding arginine while stirring to obtain a final molar ratio of 1:1. The mixture was stirred until complete dissolution was obtained. After cooling down, the pH of the solution was 7.8.
C. A solution containing 10% entacapone was prepared by adding the respective amount of H₂O to entacapone (Suven Life Sciences), stirring at 30-35°C and slowly adding arginine to obtain a final molar ratio of 1:1. The mixture was stirred until complete dissolution was obtained. After cooling down, the pH of the solution was 6.9. The pH of less concentrated solutions (6%) was 7.8. After preparation, such entacapone solution can be diluted to a 2%, 3% or 4% by weight formulation.
   Entacapone did not dissolve (at concentrations >1%) with other amino acids such as histidine and glutamic acid or in buffers at various pHs.
D. A 7% levodopa/2% carbidopa solution was prepared by adding pre-heated 0.1% Na-bisulfite solution to arginine. Levodopa was added to obtain a final molar ratio of 1:2 LD:Arg. The mixture was stirred at 75-80°C until complete dissolution was obtained. After cooling down to 60°C, carbidopa and arginine were added to obtain a final molar ratio of 1:1.2 CD(carbidopa):Arg(arginine). The mixture was stirred at 60°C until complete dissolution was obtained. After cooling, about 12.5% more arginine was added to the solution. The pH of the solution was about 9.2.
E. A 7% weight percent levodopa solution was prepared by adding pre-heated 0.1% Na-bisulfite solution to arginine. Levodopa was added to obtain a final molar ratio of 1:2 LD:Arg. The mixture was stirred at 75-80°C until complete dissolution was obtained. After cooling down, the pH of the solution was about 9.4.
F. A 2% or 4% entacapone or tolcapone solution was prepared by dissolving entacapone or tolcapone in a solution containing 1 equivalent of meglumine (molar ratio of entacapone/tolcapone:meglumine was 1:1) at a pH of 8.23.

### Example 2. Formulation preparation procedure

Levodopa (LD) and carbidopa (CD) formulations can be prepared as follows. However, as shown in Table A, the method of preparation has significant impact on the resulting physical and chemical stability of the composition.

***Method 1 (L-Ars solution).*** L-Arg and Na-Bis (Na-bisulfate) were dissolved in water. The solution was added to the LD and CD powders. The mixture was heated with stirring for 13 min at 75°C until fully dissolved. LD/CD solution was kept at RT for 10 min to cool down.

***Method 2 (all powders together).*** All powders (LD, CD and L-Arg) were weighed and water with Na-Bis was added. Suspension was heated with stirring for 13 min at 75°C until fully dissolved. LD/CD solution was kept at RT for 10 min to cool down.

***Method 3 (same as 2 without Na-Bis pre-heating).*** All powders (LD, CD and L-Arg) were weighed together and water was added. Suspension was heated with stirring for 13 min at 75°C until fully dissolved. LD/CD solution was kept at RT for 10 min to cool down.

***Method 4 (preparation in steps).*** LD and the respective amount of L-Arg were weighed; water and Na-Bis solution were added. The suspension was heated for 7 min at 75°C until fully dissolved followed by 7 min at RT. CD and the respective amount of L-Arg were weighed, and added to the LD/Arg solution at 60°C until fully dissolved. Finally, extra L-Arg was added.

***Method 5 (same as 4 without Na-Bis pre-heating).*** LD and the respective amount of L-Arg were weighed; water was added. The suspension was heated for 7 min at 75°C until fully dissolved followed by 7 min at RT. CD and the respective amount of L-Arg were weighed, and added to the LD/Arg solution at 60°C until fully dissolved. Finally, extra L-Arg was added.

After cooling down, all formulations from all methods were divided in to 3 vials, and water, Na-Bis solution or Na-Bis-Arg solution was added to each vial. The physical and chemical stability were evaluated and are presented in Tables A1 and A2.

**Table A1 - Physical stability**

| Method | | First test | | | Second test | |
|---|---|---|---|---|---|---|
| | | 24 hours | 48 hours | 72 hours | 24 hours | 48 hours |
| 1 | Water | +++ | NR | NR | ++ | NR |
| | Na-Bis solution | +++ | | | ++ | |
| | Na-Bis solution titrated with L-Arg | +++ | | | ++ | |
| 2 | Water | + | ++ | NR | - | +/- |
| | Na-Bis solution | - | + | | - | +/- |
| | Na-Bis solution titrated with L-Arg | - | +/- | | - | +/- |
| 3 | Water | - | - | + | - | Very few particles at the bottom |
| | Na-Bis solution | - | - | + (more than 13,15) | - | |
| | Na-Bis solution titrated with L-Arg | | - | + | | |
| 4 | Water | + | NR | NR | + | NR |
| | Na-Bis solution | + | | | + | |
| | Na-Bis solution titrated with L-Arg | +/- | | | + | |
| 5 | Water | ++ | NR | NR | + | NR |
| | Na-Bis solution | ++ | | | + | |
| | Na-Bis solution titrated with L-Arg | ++ | | | + | |

| | | | | | | |
|---|---|---|---|---|---|---|
| - No precipitate + Precipitate | | | | | | |

The formulations were sampled for HPLC analysis at the end of the preparation and after 5 days at RT. The recovery after 5 days at RT was calculated and compared to T=0.

The results in Table A1 and A2 clearly show that the method of formulation preparation has a significant impact on its physical and chemical stability. The formulation of Method 3 shows significantly more stability.

**Table A2 - Chemical stability**

| Method | | First test | | Second test | |
|---|---|---|---|---|---|
| | | LD recovery after 5 days (%) | CD recovery after 5 days (%) | LD recovery after 5 days (%) | CD recovery after 5 days (%) |
| 1 | Water | 90.6 | 98.0 | 89.5 | 100.4 |
| | Na-Bis solution | 90.6 | 98.6 | 87.0 | 101.3 |
| | Na-Bis solution titrated with L-Arg | 90.8 | 98.0 | 88.9 | 99.9 |
| 2 | Water | 98.4 | 98.2 | 99.1 | 100.1 |
| | Na-Bis solution | 98.2 | 98.1 | 99.4 | 100.5 |
| | Na-Bis solution titrated with L-Arg | 99.0 | 98.5 | 98.9 | 99.5 |
| 3 | Water | 99.7 | 97.5 | 95.5^{[a]} | 96.5 |
| | Na-Bis solution | 99.2 | 97.7 | 97.7^{[b]} | 99.1 |
| | Na-Bis solution titrated with L-Arg | 99.5 | 98.1 | 94.9^{[b]} | 96.2 |
| 4 | Water | 97.7 | 97.5 | 96.3 | 99.3 |
| | Na-Bis solution | 96.0 | 95.8 | 94.9 | 97.6 |
| | Na-Bis solution titrated with L-Arg | 97.7 | 97.9 | 96.3 | 100.0 |
| 5 | Water | 97.9 | 96.3 | 98.1 | 100.9 |
| | Na-Bis solution | 98.2 | 98.0 | 98.2 | 102.2 |
| | Na-Bis solution titrated with L-Arg | 97.4 | 96.7 | 98.3 | 100.6 |

| | | | | | |
|---|---|---|---|---|---|
| ^{[a]} The recovery values were lower at the second test compared to the first test, due to technical problem which occurred during the sampling. ^{[b]} The recovery values were lower at the second test compared to the first test, due to technical problem which occurred during the sampling. | | | | | |

### Example 3. Effect of arginine on long term stability of levodopa and levodopa/carbidopa compositions

Liquid formulations with levodopa, carbidopa and arginine were prepared using the procedure outlined in Example 2, and comparative studies on formulations with a different concentration of arginine and/or an amino sugar (e.g., meglumine), and /or a sugar (e.g. dextrose), and/or a base (NaOH), or another basic amino acid (e.g., lysine, histidine) were prepared. The results are shown in Table B.

**Table B**

| LD/CD Conc. (%) | Amino Acid (AA) | | | Other | | | Dissolution | Physical stability at RT |
|---|---|---|---|---|---|---|---|---|
| | Name | Conc. (%) | Molar ratio (API:Arg) | Name | Conc. (%) | Molar ratio (API:CI) | | |
| 10/0 | Lys | 8.5 | 1:2.5 | - | - | - | No | NA |
| 5/0 | Lys | 9.25 | 1:2.5 | - | - | - | No | NA |
| 3.3/0 | Lys | 6.2 | 1:2.5 | - | - | - | No | NA |
| 3/0 | Lys | 5.6 | 1:2.5 | - | - | - | Partial | NA |
| 2.5/0 | Lys | 4.6 | 1:2.5 | - | - | - | Yes | 2 days |
| 5/0 | His | 9.8 | 1:2.5 | - | - | - | No | NA |
| 2.5/0 | His | 4.9 | 1:2.5 | - | - | - | No | NA |
| 1.25/0 | His | 2.5 | 1:2.5 | - | - | - | Yes | 14 days |
| 9/0 | Arg | 8.2 | 1:1 | - | - | - | No | NA |
| 4.7/0 | Arg | 4.0 | 1:1 | - | - | - | No | NA |
| 9.5/0 | Arg | 15.9 | 1:1.9 | - | - | - | Yes | 2 days |
| 4.8/1.4 | Arg | 11.0 | 1:2.0 | - | - | - | Yes | ≥2 months |
| 4.8/1.4 | Arg | 12.1 | 1:2.2 | - | - | - | Yes | ≥2 months |
| 4.8/1.4 | Arg | 12.7 | 1:2.4 | - | - | - | Yes | ≥2 months |
| 5.4/1.5 | Arg | 13.5 | 1:2.1 | - | - | - | Yes | ≥2 months |
| 5.4/1.5 | Arg | 14.8 | 1:2.3 | - | - | - | Yes | ≥2 months |
| 6/1.5 | Arg | 14.8 | 1:2.1 | - | - | - | Yes | ≥1 month |
| 6/1.5 | Arg | 16.0 | 1:2.3 | - | - | - | Yes | ≥2 months |
| 7/2 | Arg | 17.8 | 1:2.2 | - | - | - | Yes | ≥1 month |
| 7/1.5 | Arg | 14.1 | 1:1.8 | Dex | 5.0 | - | Yes | Color change |
| 8/1.5 | Arg | 15.7 | 1:1.9 | Dex | 5.0 | - | Yes | Color change |
| 10/1.5 | Arg | 19.2 | 1:1.9 | Dex | 5.0 | - | Yes | Color change |
| 6/1.5 | Arg | 9.3 | 1:1.5 | NaOH | 4.6 | 1:0.5 | Yes | ≥3 months |
| 5/0 | - | - | - | Meg | 5.0 | 1:1 | No | NA |
| 5/0 | - | - | - | Meg | 5.9 | 1:1.2 | No | NA |
| 5/0 | - | - | - | Meg | 10.8 | 1:2.2 | Yes | NA |
| 8/1.5 | Arg | 15.7 | 1:1.9 | Meg | 3.2 | 1:0.4 | Yes | ≥4.5 months |
| 8/1.5 | Arg | 12.2 | 1:1.5 | Meg | 7.9 | 1:1 | Yes | ≥4.5 months |
| 10/1.5 | Arg | 19.2 | 1:1.9 | Meg | 4.0 | 1:0.4 | Yes | ≥4.5 months |
| 10/1.5 | Arg | 14.6 | 1:1.5 | Meg | 9.9 | 1:1 | Yes | ≥4.5 months |
| 7/1.5 | Arg | 14.1 | 1:1.9 | Meg | 2.8 | 1:0.4 | Yes | ≥4.5 months |
| 7/1.5 | Arg | 10.7 | 1:1.5 | Meg | 6.9 | 1:1 | Yes | ≥4.5 months |

| | | | | | | | | |
|---|---|---|---|---|---|---|---|---|
| *Lys - lysine; His - histidine; Arg - arginine; Dex - dextrose; Meg - meglumine; NA - not available. | | | | | | | | |

Table B indicates that arginine forms stable solutions with high concentrations of levodopa and carbidopa (>2.5%) at molar ratios <1:2.5, whereas with other basic amino acids LD does not even dissolve under these conditions. At molar ratios of LD/CD to arginine 1:<2, the solutions do not have long term stability, unless meglumine or another counterion is used, and meglumine may be used to reduce the molar ratio of arginine to LD/CD.

Liquid formulations were prepared by weighing all powders (LD, CD and L-Arg) and the addition of water pre-heated to 73±3°C. Suspension was put in a water bath at 73±3°C and stirred for 10 min until fully dissolved. LD/CD solution was kept at RT for 10 min to cool down. Then, ascorbic acid was added. Solutions were divided into glass vials and kept at +25°C and at -20°C for the indicated period of time. Prior to analyses, frozen vials were placed at RT until fully thawed. Formulations were then mixed and subjected to stability analyses.

Tables C1-C6 indicate the effect of L-Arg on physical and chemical long term stability at +25°C and at -20°C. In particular, these Tables show that there is a correlation between the molar ratio of arginine to LD/CD and stability where generally compositions having more arginine, have longer stability: LD/CD:arginine solutions (at molar ratios of 1:≥2.1) are stable for at least 1 month at RT and at -20±5°C. The solutions are stable even at very high solid concentrations (total of >45%).

**Table C1**

| Formulation | L-Arg concentration (%) | Physical stability at RT | Stability (% from T=0) at RT | | | |
|---|---|---|---|---|---|---|
| | | | 5 | | 2 months | |
| | | | LD CD | | LD CD | |
| 6/1.5% LD/CD (1% Na-Asc) | 13.5 | 6 days | 100.0 | 97.5 | | |
| | 14.2 | At least 7 days | 100.8 | 96.7 | | |
| | 14.8 | | 99.6 | 96.6 | | |
| | 16.0 | | 99.5 | 96.6 | | |
| 4.8/1.4% LD/CD (1% Na-Asc) | 11.0 | At least 2 months | 99.4 | 97.3 | 100.1 | 93.7 |
| | 11.6 | | 98.9 | 97.4 | 100.6 | 96.2 |
| | 12.1 | | 99.1 | 97.0 | 100.3 | 94.3 |
| | 12.7 | | 99.4 | 97.2 | 99.0 | 92.4 |

**Table C2**

| Formulation | L-Arg concentration (%) | Physical stability | Stability (% from T=0) 2 weeks at -20±5°C | | | |
|---|---|---|---|---|---|---|
| | | | Immediately after thawing | | 24 hours at RT | |
| | | | LD CD | | LD CD | |
| 6/1.5% LD/CD (1% Na-Asc) at -20°C | 13.5 | At least 24h after thawing | 99.7 | 98.4 | 100.0 | 99.1 |
| | 14.2 | | 99.8 | 98.1 | 101.0 | 99.4 |
| | 14.8 | | 100.0 | 98.9 | 99.9 | 98.9 |
| | 16.0 | | 99.9 | 98.8 | 100.3 | 99.3 |

**Table C3**

| Formulation | L-Arg concentration (%) | Physical stability (at RT) | |
|---|---|---|---|
| | | 1% Na-Asc | 1% Asc |
| 6/1.5% LD/CD | 14.8 | At least 3 weeks | At least 3 days |
| | 15.8 | | |
| | 16.8 | | |
| 5.4/1.5% LD/CD | 12.3 | At least 3 days | |
| | 13.5 | | |
| | 14.8 | | |

**Table C4**

| Formulation | L-Arg conc. (%) | Physical stability (after 2 month at RT) | Stability (% from T=0) at RT | | | | | |
|---|---|---|---|---|---|---|---|---|
| | | | 1 weeks | | 2 weeks | | 1 month | |
| | | | LD CD | | LD CD | | LD CD | |
| 5.4/1.5% LD/CD (1% Asc) | 13.5 | + | 101.4 | 100.4 | 101.7 | 98.4 | 98.8 | 103.1 |
| | 14.8 | + | 101.4 | 101.4 | 102.0 | 100.1 | 99.0 | 104.2 |
| 6/1.5% LD/CD (1% Asc) | 14.8 | + | 101.8 | 101.5 | 101.6 | 99.6 | 99.0 | 104.2 |
| | 16.0 | - | 101.1 | 100.4 | 102.8 | 100.6 | 99.4 | 104.2 |
| 7/2% LD/CD (1% Asc) | 17.8 | + | 101.7 | 101.0 | 102.7 | 99.7 | 98.7 | 103.1 |
| 7/2% LD/CD (1% Na-Asc) | | - | 100.6 | NA | 101.9 | 99.2 | 98.4 | 103.6 |

**Table C5**

| Formulation | L-Arg conc. (%) | Physical stability (11 days after thawing) | Stability (% from T=0) 2 weeks at -20±5°C, immediately after thawing | | Stability (% from T=0) 5 weeks at -20±5°C, immediately after thawing | |
|---|---|---|---|---|---|---|
| | | | LD CD | | LD CD | |
| 5.4/1.5% LD/CD (1% Asc) | 13.5 | + | 102.3 | 99.5 | 99.4 | 104.3 |
| | 14.8 | - | 102.7 | 101.3 | 99.6 | 104.6 |
| 6/1.5% LD/CD (1% Asc) | 14.8 | - | 102.6 | 101.1 | 99.1 | 104.2 |
| | 16.0 | - | 103.2 | 100.9 | 99.2 | 104.3 |
| 7/2% LD/CD (1% Asc) | 17.8 | + | 102.8 | 101.0 | 99.2 | 104.3 |
| 7/2% LD/CD (1% Na-Asc) | | - | 102.9 | 101.0 | 99.4 | 104.4 |

**Table C6**

| LD/CD concentration | L-Arg concentration (%) | Physical stability at 25°C |
|---|---|---|
| 12/3 % | 24.4 | Considerable precipitate on Day 5 |
| | 29.6 | Slight precipitate on Day 5 |
| | 32.1 | No precipitate on Day 7 |

Formulations containing 6/1.5% and 5.4/1.5% LD/CD and varying L-Arg concentrations were titrated with acetic acid (100%) or lactic acid (85%), to study the effect of pH and L-Arg concentration on the physical stability of the solutions (Table D).

**Table D**

| | L-Arginine (%) | Asc/ Na-Asc | pH before | Lactic (%) | pH after lactic | pH drop | 4 hours | 24 hours | | |
|---|---|---|---|---|---|---|---|---|---|---|
| | 14.8 | Na-Asc | 9.53 | 1.1 | 9.25 | -0.28 | OK | + | | |
| | | | 9.53 | 1.7 | 9.16 | -0.37 | + | + | | |
| | | | 9.53 | 2.3 | 9.02 | -0.51 | ++ | + | | |
| | 14.8 | Asc | 9.41 | 0.85 | 9.24 | -0.17 | OK | + | | |
| | | | 9.42 | 1.3 | 9.14 | -0.28 | + | + | | |
| | | | 9.41 | 1.7 | 9.06 | -0.35 | + | + | | |
| | 15.8 | Na-Asc | 9.52 | 1.1 | 9.33 | -0.19 | OK | OK | | |
| | | | 9.50 | 1.7 | 9.21 | -0.32 | OK | + | | |
| 6/1.5% LD/CD | | | 9.53 | 2.3 | 9.08 | -0.45 | + | + | | |
| | 15.8 | Asc | 9.44 | 0.85 | 9.27 | -0.17 | OK | OK | | |
| | | | 9.45 | 1.3 | 9.19 | -0.26 | OK | + | | |
| | | | 9.45 | 1.7 | 9.11 | -0.34 | + | + | | |
| | 16.8 | Na-Asc | 9.56 | 1.1 | 9.36 | -0.20 | OK | OK | | |
| | | | 9.56 | 1.7 | 9.23 | -0.33 | OK | OK | | |
| | | | 9.56 | 2.3 | 9.09 | -0.47 | OK | + | | |
| | 16.8 | Asc | 9.46 | 0.85 | 9.30 | -0.16 | OK | OK | | |
| | | | 9.46 | 1.3 | 9.20 | -0.26 | OK | OK | | |
| | | | 9.47 | 1.7 | 9.11 | -0.36 | OK | + | | |

| | L-Arginine (%) | Asc/ Na-Asc | pH before | Lactic (%) | Acetic (%) | pH after | pH drop | 2 days | 3 days | 10 days |
|---|---|---|---|---|---|---|---|---|---|---|
| | 12.3 | Na-Asc | 9.41 | 0.36 | - | 9.35 | -0.06 | OK | + | + |
| | | | 9.43 | 1.0 | - | 9.18 | -0.25 | ++ | + | + |
| | | | 9.43 | - | 0.35 | 9.29 | -0.14 | OK | + | + |
| | 12.3 | Asc | 9.28 | 0.36 | - | 9.20 | -0.08 | ++ | + | + |
| | | | 9.29 | 1.0 | - | 9.05 | -0.24 | ++ | ++ | ++ |
| | | | 9.29 | - | 0.35 | 9.14 | -0.15 | ++ | ++ | ++ |
| | 13.5 | Na-Asc | 9.50 | 0.36 | - | 9.38 | -0.12 | OK | OK | OK |
| | | | 9.48 | 1.0 | - | 9.25 | -0.23 | + | + | + |
| 5.4/1.5% LD/CD | | | 9.49 | - | 0.35 | 9.35 | -0.14 | OK | OK | OK |
| | 13.5 | Asc | 9.32 | 0.36 | - | 9.25 | -0.07 | + | + | + |
| | | | 9.33 | 1.0 | - | 9.11 | -0.22 | ++ | ++ | ++ |
| | | | 9.34 | - | 0.35 | 9.20 | -0.14 | + | + | + |
| | 14.8 | Na-Asc | 9.51 | 0.36 | - | 9.43 | -0.08 | OK | OK | OK |
| | | | 9.51 | 1.0 | - | 9.28 | -0.23 | OK | OK | OK |
| | | | 9.51 | - | 0.35 | 9.38 | -0.13 | OK | OK | OK |
| | 14.8 | Asc | 9.36 | 0.36 | - | 9.29 | -0.07 | OK | OK | OK |
| | | | 9.37 | 1.0 | - | 9.13 | -0.24 | +/- | + | + |
| | | | 9.36 | - | 0.35 | 9.23 | -0.13 | OK | OK | OK |

| | | | | | | | | | | |
|---|---|---|---|---|---|---|---|---|---|---|
| *OK - no precipitate; +/- very few particles; + slight precipitate; ++ considerable precipitate | | | | | | | | | | |

Table D indicates that ascorbic acid reduces the pH by 0.1-0.15 units as compared to Na-ascorbate and that other organic acids can further reduce the pH of the formulations. But the physical stability test results indicate that formulations are not generally stable at pH <9.15±0.5. Formulations with Na-ascorbate appear more stable than formulations with ascorbic acid at a given L-argininc concentration. Thus, it is suggested that excess of acid may cause precipitation in the absence of adequate amount of L-Arg.

Table E shows the physical and chemical stability 3 weeks post-preparation of the 6/1.5/14.8% LD/CD/Arg formulation used for the stability tests shown in Table D.

**Table E**

| Formulation | Asc/Na-Asc (1%) | Physical stability (at RT) | Stability (% of To) | |
|---|---|---|---|---|
| | | | LD | CD |
| 6/1.5% LD/CD, 14.8% L-Arg | Asc | ≥3 weeks | 103.1 | 98.9 |
| | Na-Asc | | 101.1 | 97.4 |

### Example 4: Stability of levodopa formulations with carbidopa in-vitro and ex-vivo

The effect of carbidopa on levodopa formulations was investigated. Levodopa (LD) formulations were prepared with 0, 0.5, 1, 1.5 and 2% by weight carbidopa (CD) and a constant concentration of arginine. Physical and chemical stabilities were evaluated, as shown in Table F.

**Table F**

| Formulation | | N2 +/- | Physical stability | Stability (% from T=0) | | | |
|---|---|---|---|---|---|---|---|
| | | | | 3 days | | 15 days | |
| | | | | LD | CD | LD | CD |
| 7% LD | w/o CD | + | Stable | 99.2 | NA | 103.4 | NA |
| | | - | Stable | 98.1 | NA | - | NA |
| | 0.5% CD | + | Stable | 98.6 | 94.7 | 104.1 | 108.1 |
| | | - | Stable | 98.7 | 95.6 | - | - |
| | 1% CD | + | Stable | 98.9 | 95.2 | 102.5 | 104.4 |
| | | - | Slight precipitate | 97.9 | 94.0 | - | - |
| | 1.5% CD | + | 7 days | 98.1 | 94.2 | 103.7 | 104.8 |
| | | - | | 99.6 | 96.0 | - | - |
| | 2% CD | + | 4 days | 98.9 | 94.5 | 102.9 | 103.3 |
| | | - | | 98.3 | 94.8 | - | - |

The experimental results shown in **Fig. 1A** indicate that carbidopa prevented dark yellow color formation in the presence of air, in a dose related manner. In the absence of air (with N₂ in the head space) 0.5% CD was sufficient to inhibit this color formation. It is suggested that CD inhibits oxidation of LD *in vitro.* The experimental results shown in Table F indicate that carbidopa does not have a significant effect on the chemical stability of levodopa. It also shows that the ratio between arginine and the total active ingredients is important to prevent precipitation, i.e., the physical stability of the formulation is depended on the relative concentration of arginine

In an additional experiment, LD formulations were prepared with 0, 0.5, 1 and 2% CD and respective concentrations of arginine. Physical and chemical stability were evaluated, and results are shown in Table G.

**Table G**

| Formulation | L-Arg (%) | Chemical stability at RT (% of t₀) | | | | Physical stability at RT 1 month after thawing LD |
|---|---|---|---|---|---|---|
| | | 3 days | | 1 month after thawing | | |
| | | LD | CD | LD | CD | |
| 6% LD / 0% CD | 13.5 | 102.3 | - | 6%LD / 0%CD | 13.5 | 102.3 |
| 6% LD / 0.5% CD | 14.2 | 103.3 | 100.4 | 6%LD / 0.5%CD | 14.2 | 103.3 |
| 6% LD/ 1% CD | 14.8 | 103.5 | 101.3 | 6%LD / 1%CD | 14.8 | 103.5 |
| 6% LD / 2% CD | 16.5 | 103.3 | 101.6 | 6%LD / 2%CD | 16.5 | 103.3 |

In the presence of adequate concentrations of L-arginine, all formulations *ex-vivo* were stable for at least a month at RT following thawing, as shown in Table G.

The effect of carbidopa on the stability of levodopa formulations is shown in **Fig. 1****.** A 7% LD/arginine solution, with or without 2% CD, was continuously administered at 0.08 ml/h ×18h, 37°C into a 5×5cm fresh, full-thickness pig skin. The right hand side of **Fig. 1** indicates the lack of black by-products formation, suggesting that CD inhibits oxidation of LD *ex vivo* and may also inhibit the formation of o-quinones and melanin.

### Example 5. Stability of carbidopa formulations with levodopa

The effect of levodopa on the stability of carbidopa was investigated. Table H indicates results.

Table H indicates that CD was less sensitive to oxidation and degradation and was more stable in the presence of LD: the area of impurities at retention time (R.T.) 4.82, 5.65, 12.7, 13.53 and 14.55 were significantly increased under aerobic conditions when LD was not present, and the area of impurities at R.T. at 4.82 and 13.53 were increased even in the absence of oxygen. It appears that LD may protect CD from degradation.

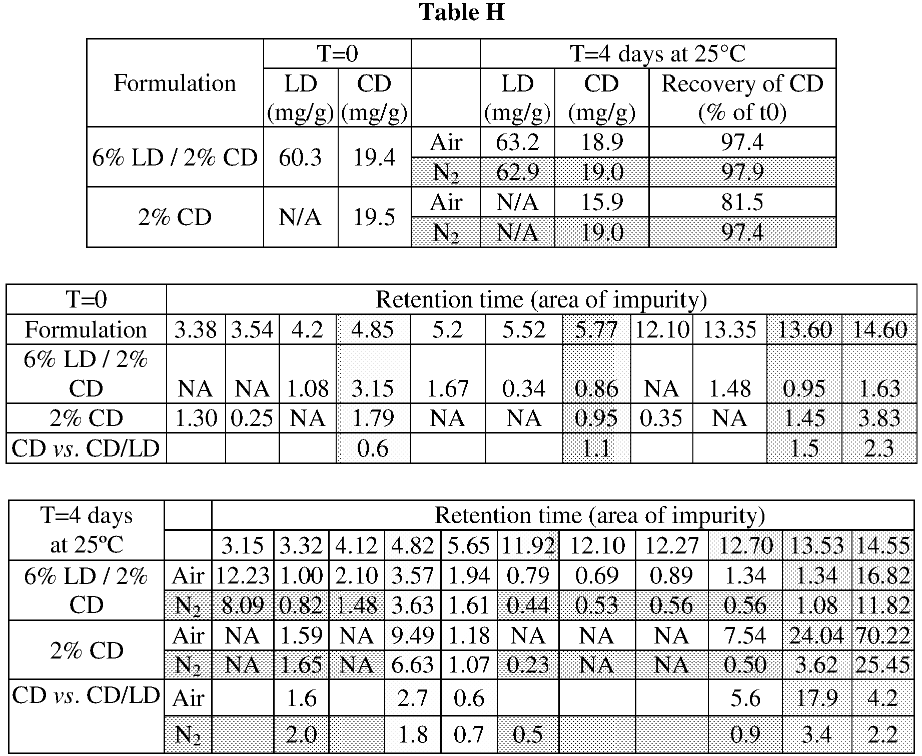

### Example 6. Toxicity and pharmacokinetics of levodopa formulations with carbidopa

The effect of carbidopa on levodopa local toxicity was investigated in pigs: solutions containing 6% LD and 0, 0.5 or 1% CD with the respective amount of arginine (13.5, 14.2 or 14.8%, respectively) were continuously administered SC to pigs at 0.16 ml/h ×24h. Each formulation was administered to 2 pigs. Skin samples were collected 8±1 days thereafter. **Fig. 2** shows that the presence of 1% carbidopa reduces the severity and extent of levodopa dependent toxicity, *in-vivo.*

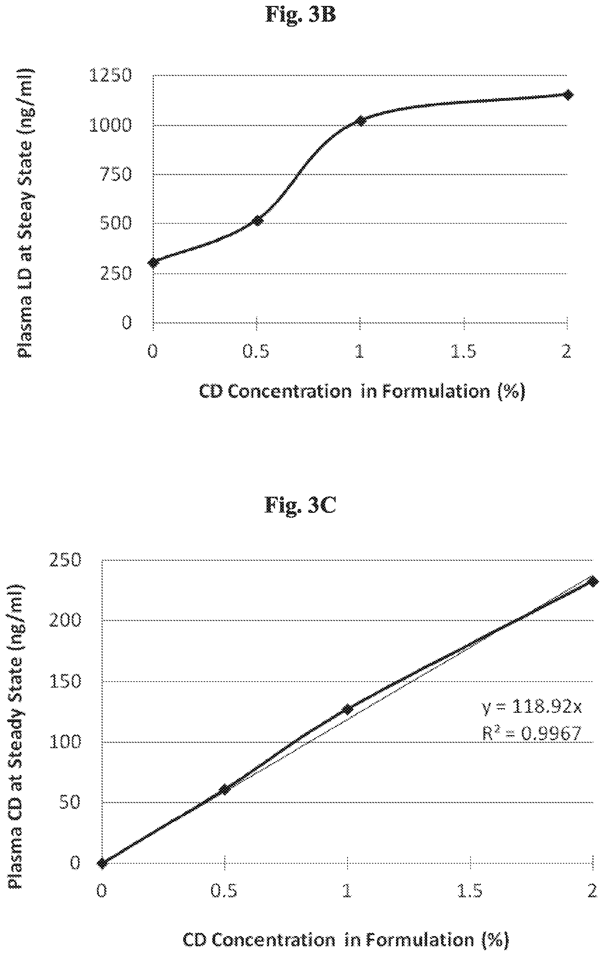

The effect of carbidopa on the pharmacokinetics of levodopa and carbidopa were investigated. Solutions containing 6% LD and 0, 0.5, 1 or 2% CD and the respective amount of arginine (13.5, 14.2, 14.8 or 16.5%, respectively) were continuously administered SC to pigs at 0.16 ml/h ×24h. **Fig. 3** shows that CD has a significant effect on the pharmacokinetics of LD. This effect was dose dependent and linear between ±0.3 and ±1.2% CD.

### Example 7. (Reference) Plasma levels of levodopa following subcutaneous administration

In this experiment, the purpose was to determine the plasma levels of LD (levodopa) following continuous subcutaneous administration of carbidopa, levodopa or entacapone and combinations thereof with oral LD/CD in pigs.

Landrace × Large White female pigs weighing about 22 kg were treated, starting on Day 1 at 15:00 as per Table I, with oral LD/CD 100/25 and with the respective test formulations, containing carbidopa, levodopa or entacapone and combinations thereof, formulated with arginine, as described above, and administered continuously subcutaneously via a dermal patch (Omnipod®) at a rate of 0.08 ml/h.

Table I indicates the treatment protocol of each group. The formulations were prepared as in Example 1 and 2.

**Table I**

| Treatment group | None | CD | CD+E | E | LD+CD | LD |
|---|---|---|---|---|---|---|
| *n* | *3* | *3* | *3* | *2* | *2* | *1* |
| SC route of administration | No SC treatment | 2% carbidopa | 2% carbidopa + 10% entacapone | 10% entacapone | 7% levodopa + 2% carbidopa | 7% levodopa |
| Oral treatment | 100/25 levodopa/carbidopa | | | | | |

Blood samples were collected following the 3^{rd} oral dose at pre-determined time points and plasma levels of levodopa, carbidopa and 3-OMD were analyzed by HPLC-ECD.

**Fig. 4** indicates the mean levodopa plasma concentrations following oral administration of Sinemet (oral 100/25 LD/CD) with continuous SC administration of entacapone (200 mg/24h) ±CD (40mg/24h), as two separate compositions (**Fig. 4A**); or LD (140 mg/24h) ±CD (40mg/24h) in pigs (**Fig. 4B**) (all subcutaneous formulations included arginine, as above).

Results show that there is a synergistic effect between entacapone (200 mg/24h) and CD (40mg/24h) on the plasma pharmacokinetics (PK) of levodopa (ng/ml) when co-administered continuously subcutaneously, as compared to the calculated LD plasma PK obtained after adding the plasma concentrations of LD following the continuous SC administration of CD and entacapone each alone (**Fig. 1A** and Table 2, C vs. B+D). Results also show that there is an additive effect between levodopa (140 mg/24h) and CD (40mg/24h) on the plasma PK of levodopa (ng/ml) when co-administered continuously subcutaneously, as compared to the calculated LD plasma PK obtained after adding the plasma concentrations of LD following the continuous SC administration of CD and LD each alone (**Fig. 1B** and Table 2, E *vs.* D+F). Moreover, the results suggest that continuous SC administration of LD and CD may be sufficient to maintain constant, continuous levodopa plasma concentrations even in the absence of oral LD/CD administration (**Fig. 4B** dotted line and Table J 'E minus A'). Table J presents concentrations of plasma levodopa 6½ and 8h post-oral LD/CD administration.

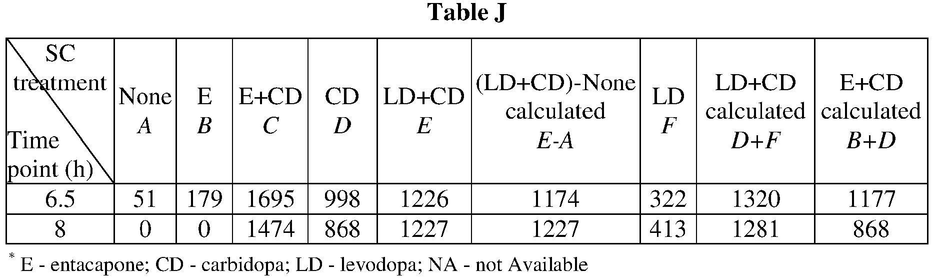

**Fig. 5** shows tissue biopsies from the application site of the levodopa-carbidopa arginine combination formulation and the levodopa/arginine formulation. No visible tissue irritation or damage was apparent in the levodopa-carbidopa arginine formulation. The site administered with levodopa-arginine formulation appears to have some blackening of tissue. Without being limited by any theory, it is thought that having carbidopa and arginine together with levodopa (arginine) formulation protects the local tissue from local damage of levodopa by preventing oxidation of levodopa into irritant by products, and that carbidopa is a potent anti-oxidant.

### Example 8. Additional exemplary carbidopa and levodopa/carbidopa formulations

In Tables K and L, additional exemplary carbidopa and levodopa/carbidopa formulations are provided.

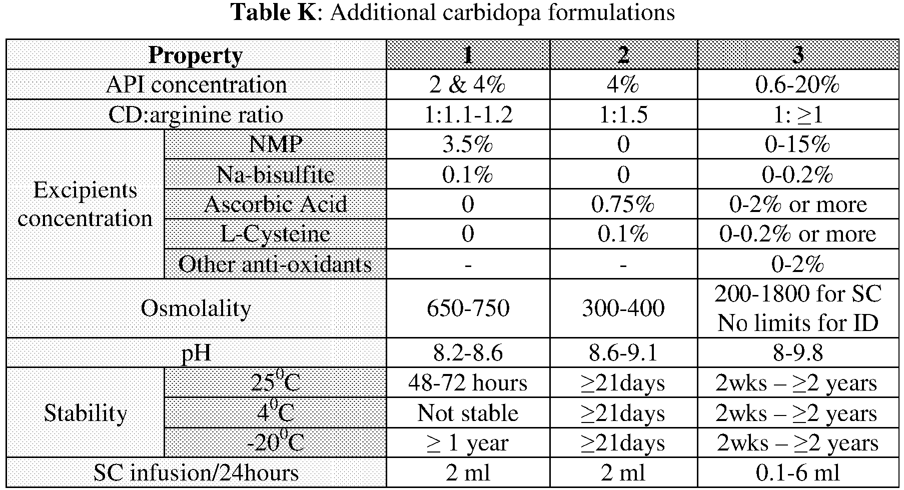

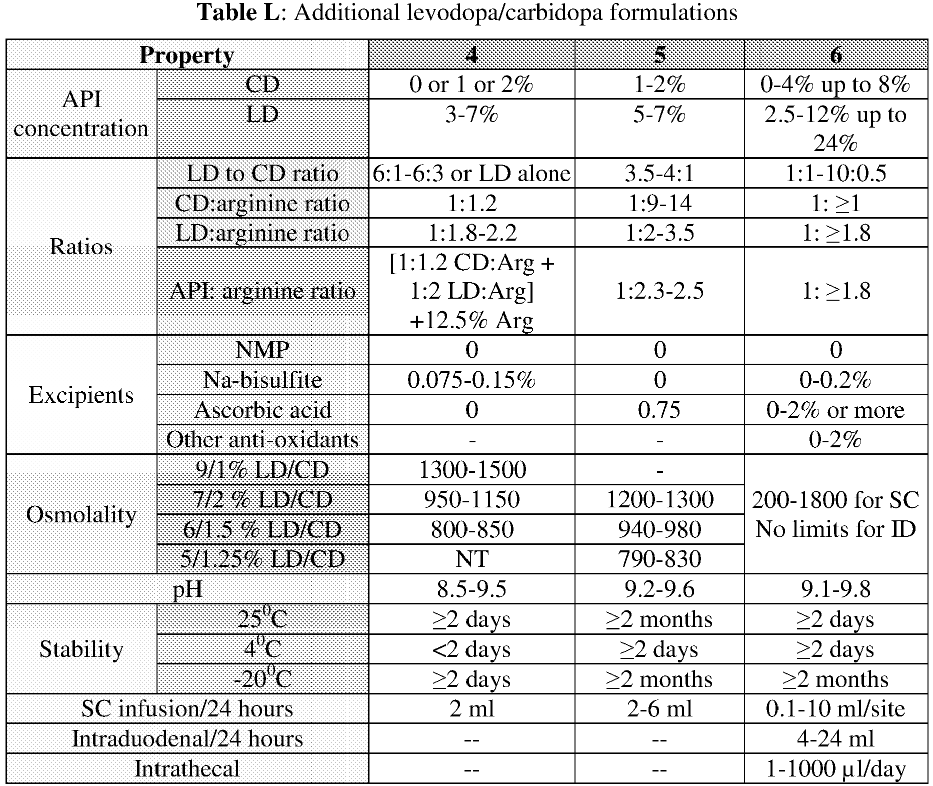

### Example 9. Plasma levels of levodopa, carbidopa, and 3-O-methyldopa following continuous subcutaneous administration of carbidopa and levodopa and oral administration of entacapone

In this experiment, the purpose was to determine the plasma levels of levodopa (LD), carbidopa (CD), and 3-O-methyldopa (3-OMD) following continuous subcutaneous administration of carbidopa and levodopa, and oral administration of entacapone in human volunteers.

A single center, double-blind, randomized, placebo-controlled, study was conducted with six healthy Caucasian males volunteers, aged 18-40 years old. LD (6%) /CD (1.5%) was administered at a rate of 240 µl/h, corresponding to 360 mg of LD and 90 mg of CD per 24 hours. Entacapone (200 mg) was administered orally every 2 hours, starting 15h after infusion initiation of LD/CD. Plasma LD, CD and 3-OMD concentrations were quantified at pre-determined time points.

As shown in **Fig. 6**, the results demonstrate that oral entacapone increased the plasma concentrations of LD attainable with the continuous SC administration of LD/CD by 50% within 9 hours after the initiation of entacapone dosing. The plasma concentrations of LD did not reach a steady state by the time the study was terminated 24 hours after LD/CD infusion initiation and 9 hours after entacapone dosing initiation. The plasma concentrations of 3-OMD were significantly reduced. These results suggest that rather than administering 200 mg of entacapone 6-8 times daily, as is typically done with oral LD/CD administration, 400 mg entacapone can instead be administered, e.g., twice or three times daily.

## Claims

1. Carbidopa, levodopa and entacapone for use as a combination in the treatment of Parkinson's disease, wherein the carbidopa and the levodopa are formulated as a sole parenteral composition for continuous subcutaneous administration, and said entacapone is formulated as an oral composition and is administered at a dose of 400, 450, 500, 550 or 600 mg twice or three times a day.

2. The carbidopa, levodopa and entacapone for use according to claim 1, wherein said entacapone is administered at a dose of 400 mg twice or three times a day.

3. The carbidopa, levodopa and entacapone for use according to claim 1 or 2, wherein said entacapone is administered twice a day.

4. The carbidopa, levodopa and entacapone for use according to any one of claims 1 to 3, wherein said parenteral composition further comprises arginine.

5. The carbidopa, levodopa and entacapone for use according to claim 4, wherein said parenteral composition has a molar ratio of carbidopa and levodopa to arginine of 1:2 to 1:3.5.

6. The carbidopa, levodopa and entacapone for use according to claim 5, wherein said parenteral composition comprises (i) arginine, 0.1% to 2% by weight carbidopa, and 4% to 8% by weight levodopa; or (ii) arginine, 0.6% to 1.5% by weight carbidopa, and 6% by weight levodopa.

7. The carbidopa, levodopa and entacapone for use according to claim 6, wherein said use comprises administering said parenteral composition at a rate of 0.1 to 1000 µl/h/site; or at a volume of 2 to 10 ml/24h/site; or at a dose of 80 to 800 mg levodopa/day and 20 to 200 mg carbidopa/day; or at a rate of 240 to 360 mg levodopa and 60 to 90 mg carbidopa/day/site.

8. The carbidopa, levodopa and entacapone for use according to claim 7, wherein said use comprises administering said parenteral composition at a volume of 4 to 6 ml/24h/site.

9. The carbidopa, levodopa and entacapone for use according to claim 5, wherein said parenteral composition comprises (i) arginine, 1% to 4% by weight carbidopa, and 6% to 16% by weight levodopa; or (iv) arginine, 1.5% to 2.5% by weight carbidopa, and 12% by weight levodopa.

10. The carbidopa, levodopa and entacapone for use according to claim 9, wherein said use comprises administering said parenteral composition at a rate of 0.2 to 2000 µl/h/site; or at a volume of 10 to 24 ml/24h/site; or at a dose of 600 to 4000 mg levodopa/day and 60 to 500 mg carbidopa/day; or at a rate of 800 to 1600 mg levodopa and 200 to 400 mg carbidopa/ day/site.

11. The carbidopa, levodopa and entacapone for use according to claim 10, wherein said use comprises administering said parenteral composition at a volume of 12 to 16 ml/24h/site.

## Patentansprüche

1. Carbidopa, Levodopa und Entacapon zur Verwendung als eine Kombination bei der Behandlung der Parkinson-Krankheit, wobei das Carbidopa und das Levodopa als eigenständige parenterale Zusammensetzung zur kontinuierlichen subkutanen Verabreichung formuliert sind, und besagtes Entacapon als eine orale Zusammensetzung formuliert ist und in einer Dosis von 400, 450, 500, 550 oder 600 mg zweimal oder dreimal am Tag verabreicht wird.

2. Carbidopa, Levodopa und Entacapon zur Verwendung gemäß Anspruch 1, wobei besagtes Entacapon in einer Dosis von 400 mg zweimal oder dreimal am Tag verabreicht wird.

3. Carbidopa, Levodopa und Entacapon zur Verwendung gemäß Anspruch 1 oder 2, wobei besagtes Entacapon zweimal am Tag verabreicht wird.

4. Carbidopa, Levodopa und Entacapon zur Verwendung gemäß einem der Ansprüche 1 bis 3, wobei besagte parenterale Zusammensetzung ferner Arginin umfasst.

5. Carbidopa, Levodopa und Entacapon zur Verwendung gemäß Anspruch 4, wobei besagte parenterale Zusammensetzung ein molares Verhältnis von Carbidopa und Levodopa zu Arginin von 1:2 bis 1:3,5 aufweist.

6. Carbidopa, Levodopa und Entacapon zur Verwendung gemäß Anspruch 5, wobei besagte parenterale Zusammensetzung (i) Arginin, 0,1 bis 2 Gew.-% Carbidopa und 4 bis 8 Gew.-% Levodopa; oder (ii) Arginin, 0,6 bis 1,5 Gew.-% Carbidopa und 6 Gew.-% Levodopa umfasst.

7. Carbidopa, Levodopa und Entacapon zur Verwendung gemäß Anspruch 6, wobei besagte Verwendung die Verabreichung besagter parenteraler Zusammensetzung in einer Rate von 0,1 bis 1000 µL/h/Stelle; oder in einem Volumen von 2 bis 10 mL/24 h/Stelle; oder in einer Dosis von 80 bis 800 mg Levodopa/Tag und 20 bis 200 mg Carbidopa/Tag; oder in einer Rate von 240 bis 360 mg Levodopa und 60 bis 90 mg Carbidopa/Tag/Stelle umfasst.

8. Carbidopa, Levodopa und Entacapon zur Verwendung gemäß Anspruch 7, wobei besagte Verwendung die Verabreichung besagter parenteraler Zusammensetzung in einem Volumen von 4 bis 6 mL /24 h/Stelle umfasst.

9. Carbidopa, Levodopa und Entacapon zur Verwendung gemäß Anspruch 5, wobei besagte parenterale Zusammensetzung (i) Arginin, 1 bis 4 Gew.-% Carbidopa und 6 bis 16 Gew.-% Levodopa; oder (iv) Arginin, 1,5 bis 2,5 Gew.-% Carbidopa und 12 Gew.-% Levodopa umfasst.

10. Carbidopa, Levodopa und Entacapon zur Verwendung gemäß Anspruch 9, wobei besagte Verwendung die Verabreichung besagter parenteraler Zusammensetzung in einer Rate von 0,2 bis 2000 µL/h/Stelle; oder in einem Volumen von 10 bis 24 mL/24 h/Stelle; oder in einer Dosis von 600 bis 4000 mg Levodopa/Tag und 60 bis 500 mg Carbidopa/Tag; oder in einer Rate von 800 bis 1600 mg Levodopa und 200 bis 400 mg Carbidopa/Tag/Stelle umfasst.

11. Carbidopa, Levodopa und Entacapon zur Verwendung gemäß Anspruch 10, wobei besagte Verwendung die Verabreichung besagter Zusammensetzung in einem Volumen von 12 bis 16 mL/24 h/Stelle umfasst.

## Revendications

1. Carbidopa, lévodopa et entacapone destinés à être utilisés dans le traitement de la maladie de Parkinson, dans lesquels la carbidopa et la lévodopa sont formulées sous la forme d'une seule composition parentérale pour une administration sous-cutanée continue et ledit entacapone est formulé sous la forme d'une composition orale et est administré à une dose de 400, 450, 500, 550 ou 600 mg deux ou trois fois par jour.

2. Carbidopa, lévodopa et entacapone destinés à être utilisés selon la revendication 1, dans lesquels ledit entacapone est administré à une dose de 400 mg deux ou trois fois par jour.

3. Carbidopa, lévodopa et entacapone destinés à être utilisés selon la revendication 1 ou 2, dans lesquels ledit entacapone est administré deux fois par jour.

4. Carbidopa, lévodopa et entacapone destinés à être utilisés selon l'une quelconque des revendications 1 à 3, dans lesquels ladite composition parentérale comprend en outre l'arginine.

5. Carbidopa, lévodopa et entacapone destinés à être utilisés selon la revendication 4, dans lesquels ladite composition parentérale présente un rapport molaire entre la carbidopa, la lévodopa et l'arginine de 1:2 à 1:3,5.

6. Carbidopa, lévodopa et entacapone destinés à être utilisés selon la revendication 5, dans lesquels ladite composition parentérale comprend (i) l'arginine, 0,1 % à 2 % en poids de carbidopa et 4 % à 8 % en poids de lévodopa ; ou (ii) l'arginine, 0,6 % à 1,5 % en poids de carbidopa et 6 % en poids de lévodopa.

7. Carbidopa, lévodopa et entacapone destinés à être utilisés selon la revendication 6, dans lesquels ladite utilisation comprend l'administration de ladite composition parentérale à un taux de 0,1 à 1000 µl/h/site ; ou à un volume de 2 à 10 ml/24 h/site ; ou à une dose de 80 à 800 mg de lévodopa/jour et 20 à 200 mg de carbidopa/jour ; ou à un taux de 240 à 360 mg de lévodopa et 60 à 90 mg de carbidopa/jour/site.

8. Carbidopa, lévodopa et entacapone destinés à être utilisés selon la revendication 7, dans lesquels ladite utilisation comprend l'administration de ladite composition parentérale à un volume de 4 à 6 ml/24 h/site.

9. Carbidopa, lévodopa et entacapone destinés à être utilisés selon la revendication 5, dans lesquels ladite composition parentérale comprend (i) l'arginine, 1% à 4 % en poids de carbidopa et 6 % à 16 % en poids de lévodopa ; ou (iv) l'arginine, 1,5 % à 2,5 % en poids de carbidopa et 12 % en poids de lévodopa.

10. Carbidopa, lévodopa et entacapone destinés à être utilisés selon la revendication 9, dans lesquels ladite utilisation comprend l'administration de ladite composition parentérale à un taux de 0,2 à 2 000 µl/h/site ; ou à un volume de 10 à 24 ml/24 h/site ; ou à une dose de 600 à 4 000 mg de lévodopa/jour et 60 à 500 mg de carbidopa/jour ; ou à un taux de 800 à 1 600 mg de lévodopa et 200 à 400 mg de carbidopa/jour/site.

11. Carbidopa, lévodopa et entacapone destinés à être utilisés selon la revendication 10, dans lesquels ladite utilisation comprend l'administration de ladite composition parentérale à un volume de 12 à 16 ml/24 h/site.
